# EUROPEAN PATENT SPECIFICATION

(11) **EP 1 140 793 B1**
(45) Date of publication and mention of the grant of the patent: **24.09.2003**
(21) Application number: 99963652.5
(22) Date of filing: 23.12.1999
(51) Int. Cl.: C07C 227/04, C07C 229/28, C07C 205/51

(54) **PROCESS FOR THE SYNTHESIS OF 1-(AMINOMETHYL)CYCLOHEXYL-ACETIC ACID**
VERFAHREN ZUR HERSTELLUNG VON 1-AMINOMETHYLCYCLOHEXANESSIGSÄURE
PROCEDE DE SYNTHESE D'ACIDE ACETIQUE 1-(AMINOMETHYL)-CYCLOHEXENE

(30) Priority: 29.12.1998 HU 9830034
(43) Date of publication of application: 10.10.2001
(73) Proprietor: RICHTER GEDEON VEGYESZETI GYAR R.T., 1103 Budapest X (HU)
(72) Inventor: GIZUR, Tibor, H-1162 Budapest (HU); LENGYEL, Zoltánné, H-1125 Budapest (HU); SZALAI, Krisztina, H-1039 Budapest (HU)
(74) Representative: HOFFMANN - EITLE
(86) International application number: HU9900102
(87) International publication number: WO00039074

(56) References cited:
- EP-A- 0 414 263
- EP-A- 0 414 274
- EP-A- 0 432 504
- WO-A-99/21824
- BRYANS J S ET AL: "Investigation into the preferred conformation of Gabapentin for interaction with its binding site on the alpha2delta subunit of a calcium channel" BIOORGANIC & MEDICINAL CHEMISTRY LETTERS,GB,OXFORD, vol. 7, no. 19, 7 October 1997 (1997-10-07), pages 2481-2484, XP004136469 ISSN: 0960-894X
- GARETH GRIFFITHS ET AL: "Novel Syntheses of Gabapentin via Addition of Hydrocyanic Acid to Cyclohexylidenemalonate or Cyano(cyclohexylidene)acetate" HELVETICA CHIMICA ACTA,CH,VERLAG HELVETICA CHIMICA ACTA. BASEL, vol. 74, no. 2, 1991, pages 309-314, XP002100736 ISSN: 0018-019X

## Description

The invention relates to a new process for the synthesis of 1-(aminomethyl)cyclohexyl-acetic acid of the formula (I) via the new intermedier 1-(nitromethyl)cyclohexyl-acetic acid derivative of general formula (II), wherein R represents hydrogen, benzyl group, diphenylmethyl group or C₁-C₄ alkyl or alkoxy aromatic ring substituted derivatives thereof.

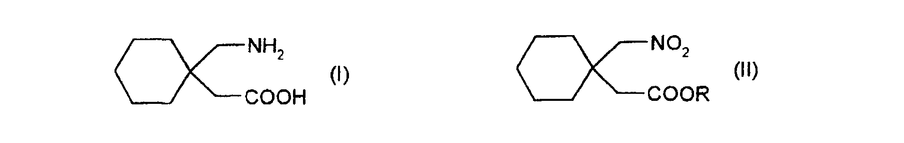

The 1-(aminomethyl)cyclohexyl-acetic acid of formula (I), otherwise known as gabapentin is the active ingredient of the GABA antagonist drug. Several methods are known from the literature for the synthesis of this compound.

In most of the known methods an intermedier is hydrolysed with acid, and gabapentin is obtained from the so formed gabapentin hydrochloride salt by using ion exchange resin. This process is described in the German patent No. DE 2 460 891, in which the 1,1-cyclohexyldiacetic acid anhydride is converted into hydroxamic acid and the latter is transformed via Lossen degradation into the hydrochloride salt of the product. The US patent No. US 4 024 175 describes a method where the same 1,1-cyclohexyldiacetic acid anhydride is used as starting material. The anhydride is first transformed into a monomethyl ester monosalt and then a monoacid monoazide is obtained from it. The gabapentin hydrochloride is prepared from the latter via Curtius degradation.

Similarly gabapentin hydrochloride is formed in the procedure described in the European patent No. EP 414 274. According to this invention the alkyl ester of 1-(nitromethyl)acetic acid is transformed into a 2-aza-spiro[4,5]decane-3-on derivative by catalytic hydrogenation. The gabapentin hydrochloride is obtained from the latter lactam derivative by refluxing it with hydrochloric acid and gabapentin is isolated by using ion-exchange resin.

The disadvantages of the above mentioned procedures are as follows. The gabapentin is obtained as its hydrochloride salt and gabapentin itself can be isolated only by using labour-demanding and expensive ion-exchange method. To avoid the unwanted lactam formation side-reaction requires also a labour-demanding and expensive technique. Further disadvantages of these procedures are the use of hazardous reagents, e.g. potassium cyanide, sodium azide and the expensive pressure resistant equipment.

The procedure described in the European patent No. EP 414 275 avoids the formation of the lactam compound and the gabapentin hydrochloride, and this way the use of the expensive ion-exchange method. According to this procedure cyano-cyclohexane-maleinic acid derivatives are hydrolysed with base, decarboxylated and finally the nitril group is catalytically hydrogenated. On the other hand this patent does not describe the synthesis of the cyano-cyclohexane-maleinic acid derivatives, which is a multi step, tedious process. It is important to note, that the synthesis of the maleinic acid ester is four steps starting from cyclohexanon, so the synthesis of gabapentin is altogether seven steps. The patent does not mention the purity of the obtained gabapentin either, in contrast to other patents, which describe the synthesis of gabapentin, e.g. EP 414 274.

The aim the invention is to elaborate an economical, industrially applicable process for the synthesis of gabapentin, which eliminates the disadvantages of the above mentioned procedures and makes possible the simple synthesis of the very pure final product of formula (I) in fewer steps and in good yield.

The synthesis of gabapentin according to the process of the invention is as follows
a) the alkyl ester of cyclohexylidene-acetic acid of general formula (VI) - wherein R₂ represents C₁-C₄ alkyl group - is transformed into the alkyl ester of 1-(nitromethyl)cyclohexyl-acetic acid of general formula (V) - wherein the meaning of R₂ is as defined above - with nitromethane in the presence of a base, the latter is hydrolysed with aqueous methanolic solution of potassium hydroxide and the obtained 1-(nitromethyl)cyclohexyl-acetic acid of formula (lla) is hydrogenated in a solvent in the presence of a catalyst to yield the desired product of formula (I), and in given case transformation of the obtained compound into a pharmaceutically acceptable salt or 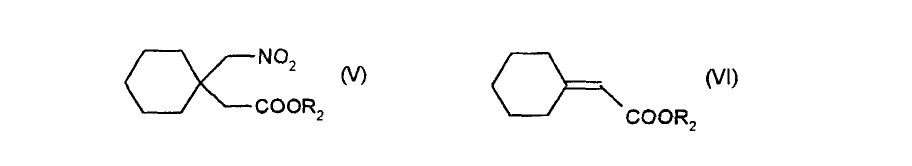 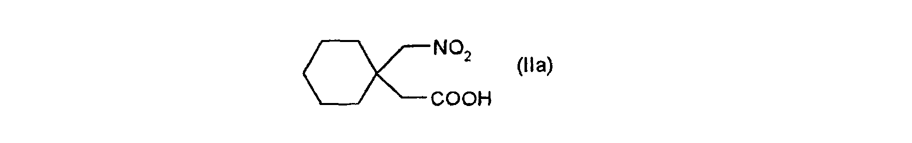
b) the alkyl ester of cyclohexylidene-acetic acid of general formula (VI) - wherein the meaning of R₂ is as defined above - is hydrolysed with aqueous methanolic solution of potassium hydroxide and the obtained cyclohexylidene-acetic acid of formula (IV) is reacted with a reagent of formula R₁-X - wherein R₁ represents benzyl group, diphenylmethyl group or in given case C₁-C₄ alkyl or alkoxy aromatic ring substituted derivatives thereof and X represents halogen atom - to give the appropriate cyclohexylidene acid derivative of general formula (III) - wherein the meaning of R₁ is as 1- defined above - and this intermedier is transformed into (nitromethyl)cyclohexyl-acetic acid derivative of general formula (IIb) - wherein the meaning of R₁ is as defined above - with nitromethane and the latter is hydrogenated in a solvent in the presence of a catalyst, and in given case transformation of the obtained compound into a pharmaceutically acceptable salt. 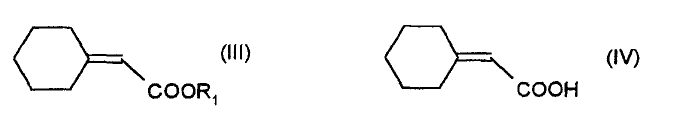 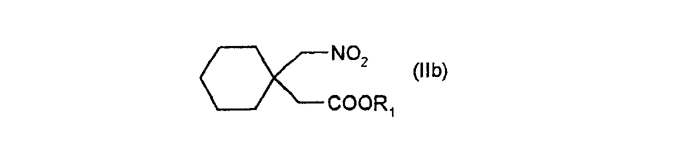
The process of the invention is illustrated on Scheme 1.

The invention based on the observation, that the reduction of the new compounds of general formula (II) at atmospheric pressure yields directly the pure desired final product.

Surprisingly it was found, that using the compounds of general formula (II) as starting materials in the reduction step the lactam compound is not formed, but the very pure gabapentin is obtained directly. This was not anticipated in the knowledge of previous procedures, as the ability of lactam formation of this type of compounds is known from the literature (e.g. EP 414 274).

The alkyl ester of cyclohexylideneacetic acid of general formula (VI) used as starting material can be prepared according to the literature via the reaction of cyclohexanone and the appropriate ester of diethylphosphono-acetic acid.

In the last hydrogenation step any catalysts can be used, which are generally applicable in hydrogenation reactions, rare metal catalysts, e.g. rhodium or palladium, Raney nickel or cobalt catalysts, in given case on a carrier e.g. on carbon, preferably palladium on activated carbon, more preferably 10% of the compound to be reduced.

The hydrogenation is carried out in an inert organic solvent, preferably in a C₁-C₄ alcohol, more preferably in methanol, at 10-50°C, under 1-20 kPa pressure, preferably at room temperature and under atmospheric pressure.

The Michael addition of the ester of cyclohexylidene-acetic acid with nitromethane is carried out in the presence of a base, preferably potassium hydroxide.

The hydrolysis of the alkyl ester group is carried out with base, preferably aqueous methanolic solution of potassium hydroxide at room temperature, than the acid is liberated with 10% aqueous potassium dihydrogenphosphate solution.

After filtration of the catalyst the product is isolated by concentration of the filtrate. The product obtained on concentration is 98-99% pure, the yield is 50-70%.
The advantages of this procedure are as follows:
- the obtained product is very pure
- the number of reaction steps is less than in the known procedures
- the lactam compound, which is very difficult to remove, is not formed
- neither special pressure resistant equipment nor expensive catalyst is needed
- the final product can be obtained without applying difficult and complicated ion-exchange technology
- no poisonous or dangerous materials are needed

### Examples

### Example 1

### a) Synthesis of 1 -(nitromethyl)cyclohexyl-acetic acid

A solution of 4.3 g (0.02 mol) of methyl 1-(nitromethyl)cyclohexyl-acetate in a mixture of 50 ml of methanol and 20 ml of 10% aqueous potassium hydroxide is stirred at room temperature for 24 h, then the methanol is distilled off in vacuo. The pH of the resulted aqueous solution is adjusted to 7 with 10% aqueous potassium dihydrogenphosphate solution. The solution is extracted three times with 30 ml of ethyl acetate, the combined organic layers are dried over sodium sulphate and concentrated to yield 3.2 g (80%) of the title compound as oil.

### b) Synthesis of 1-(aminomethyl)cyclohexyl-acetic acid

A solution of 2.01 g (0.01 mol) of 1-(nitromethyl)cyclohexyl-acetic acid in 50 ml of methanol is hydrogenated in the presence of 0.2 g of palladium on activated carbon at atmospheric pressure. The catalyst is filtered off and the filtrate is concentrated to 10 ml. 20 ml of tetrahydrofuran is added to the residue and the precipitated crystals were filtered off and dried to yield 1.3 g (80%) of the title compound. Mp:164-169°C

### Example 2

### Synthesis of 1-(aminomethyl)cyclohexyl-acetic acid

A solution of 5 g (0.017 mol) of benzyl 1-(nitromethyl)cyclohexyl-acetate in 50 ml of methanol is added to a mixture of 0.5 g of prehydrogenated palladium, 10% on activated carbon in 50 ml of methanol. This mixture is hydrogenated at room temperature under atmospheric pressure until the calculated hydrogen is consumed, then the catalyst is filtered off, the filtrate is concentrated to about 15 ml and 30 ml of tetrahydrofuran is added to precipitate the title compound. Yield: 1.5 g (51%). Mp: 168°C.

## Claims

1. Process for the synthesis of 1-(aminomethyl)cyclohexyl-acetic acid and pharmaceutically acceptable salt thereof **characterised by**
a) transformation of the alkyl ester of cyclohexylidene-acetic acid of formula (VI) - wherein R₂ represents C₁-C₄ alkyl group - into the alkyl ester of 1 -(nitromethyl)cyclohexyl-acetic acid of formula (V) - wherein the meaning of R₂ is as defined above - with nitromethane in the presence of a base, hydrolysis with aqueous methanolic solution of potassium hydroxide and hydrogenation of the obtained 1-(nitromethyl)cyclohexylacetic acid of formula (IIa) in a solvent in the presence of a catalyst and in given case transformation of the obtained compound into a pharmaceutically acceptable salt or 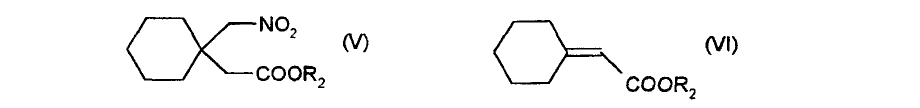 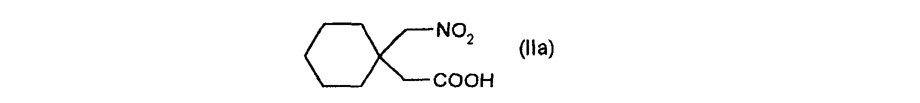
b) hydrolysis of the alkyl ester of cyclohexylidene-acetic acid of formula (VI) - wherein R₂ represents C₁-C₄ alkyl group - into the cyclohexylidene-acetic acid of formula (IV) with aqueous methanolic solution of potassium hydroxide, reaction of the obtained acid of formula (IV) with a reagent of formula R₁-X - wherein R₁ represents benzyl group, diphenylmethyl group or in given case C₁-C₄ alkyl or alkoxy aromatic ring substituted derivatives thereof and X represents halogen atom - to give the intermedier cyclohexylidene acid derivative of formula (III) - wherein the meaning of R₁ is as defined above - transformation of this intermedier into the 1 -(nitromethyl)cyclohexyl-acetic acid derivative of formula (llb) - wherein the meaning of R₁ is as defined above with nitromethane - and hydrogenation of the latter in a solvent in the presence of a catalyst and in given case transformation of the obtained compound into a pharmaceutically acceptable salt.

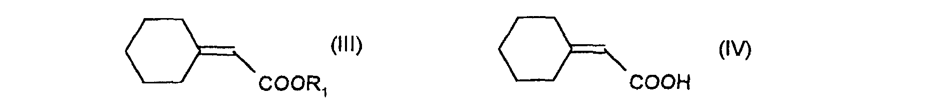

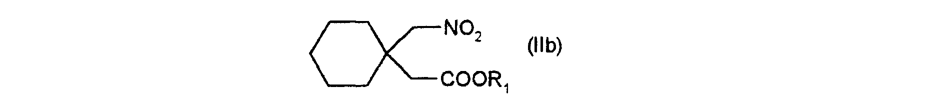

2. Process b) of claim 1 **characterised by** using benzyl halide as reagent of formula R₁-X.

3. Process b) of claim 1 **characterised by** using diphenylmethyl halide as reagent of formula R₁-X.

4. The process of claim 1-3 **characterised by** carrying out the hydrogenation in an inert organic solvent.

5. The process of claim 1-3 **characterised by** using palladium on activated carbon as catalyst.

6. The new compounds of formula (II), wherein R represents hydrogen, benzyl, diphenylmethyl group or in given case C₁-C₄ alkyl or alkoxy aromatic ring substituted derivatives thereof.

7. 1-(nitromethyl)cyclohexyl-acetic acid

8. benzyl 1-(nitromethyl)cyclohexyl-acetate

9. diphenylmethyl 1-(nitromethyl)cyclohexyl-acetate

## Patentansprüche

1. Verfahren zur Synthese von 1-(Aminomethyl)cyclohexylessigsäure und pharmazeutisch annehmbaren Salzen davon, **gekennzeichnet durch**
. a) Umwandlung des Alkylesters von Cyclohexyliden-essigsäure der Formel (VI) - wobei R₂ eine C₁-C₄-Alkyl-Gruppe darstellt - mit Nitromethan in Gegenwart einer Base in den Alkylester von 1-(Nitromethyl)cyclohexylessigsäure der Formel (V) - wobei die Bedeutung von R₂ wie oben definiert ist - Hydrolyse mit wäßriger methanolischer Lösung von Kaliumhydroxid und Hydrierung der erhaltenen l-(Nitromethyl)cyclohexyl-essigsäure der Formel (IIa) in einem Lösungsmittel in Gegenwart eines Katalysators und gegebenenfalls Umwandlung der erhaltenen Verbindung in ein pharmazeutisch annehmbares Salz oder 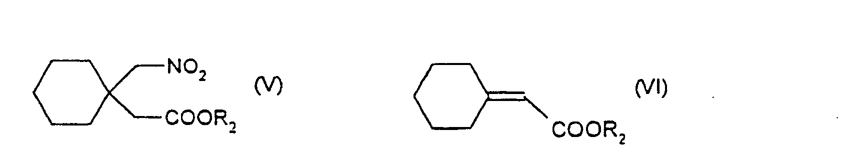 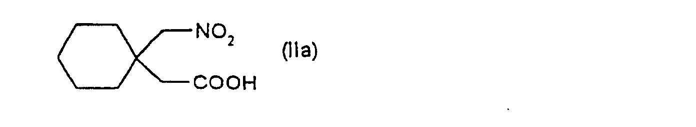
b) Hydrolyse des Alkylesters der Cyclohexyliden-essigsäure der Formel (VI) - worin R₂ eine C₁-C₄-Alkyl-Gruppe darstellt - mit wäßriger methanolischer Lösung von Kaliumhydroxid in die Cyclohexyliden-essigsäure der Formel (IV), Umsetzung der erhaltenen Säure der Formel (IV) mit einem Reagens der Formel R₁-X - worin R₁ eine Benzyl-Gruppe, Diphenylmethyl-Gruppe oder gegebenenfalls . am aromatischen Ring mit C₁-C₄-Alkyl oder Alkoxy substituierte Derivate davon darstellt und X ein Halogenatom darstellt - um das intermediäre Cyclohexylidensäure-Derivat der (III) zu ergeben - worin die Bedeutung von R₁ wie oben definiert ist - Umwandlung dieses Zwischenprodukts mit Nitromethan in das 1-(Nitromethyl)cyclohexylessigsäure-Derivat der Formel (IIb) - wobei die Bedeutung von R₁ wie oben definiert ist - und Hydrierung des letzteren in einem Lösungsmittel in Gegenwart eines Katalysators, und gegebenenfalls Umwandlung der erhaltenen Verbindung in ein pharmazeutisch annehmbares Salz.

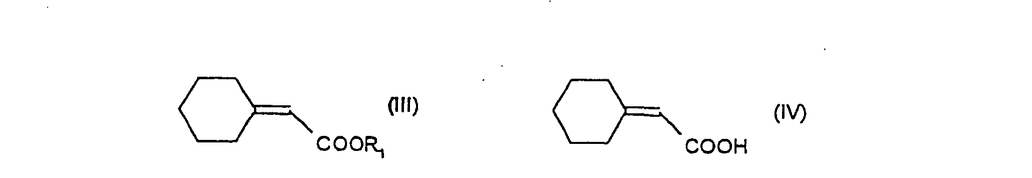

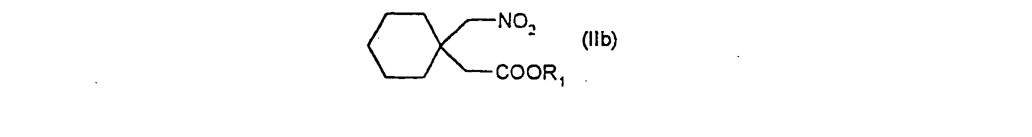

2. Verfahren b) gemäß Anspruch 1, **dadurch gekennzeichnet, daß** Benzylhalogenid als Reagens der Formel R₁-X verwendet wird.

3. Verfahren b) gemäß Anspruch 1, **dadurch gekennzeichnet, daß** Diphenylmethylhalogenid als Reagens der Formel R₁-X verwendet wird.

4. Verfahren gemäß Anspruch 1 bis 3, **dadurch gekennzeichnet, daß** die Hydrierung in einem inerten organischen Lösungsmittel durchgeführt wird.

5. Verfahren gemäß Anspruch 1 bis 3, **dadurch gekennzeichnet, daß** Palladium-auf-Aktivkole als Katalysator verwendet wird.

6. Neue Verbindungen der Formel (II), worin R Wasserstoff, eine Benzyl-, eine Diphenylmethyl-Gruppe oder gegebenenfalls am aromatischen Ring mit C₁-C₄-Alkyl oder Alkoxy substituierte Derivate davon darstellt.

7. 1-(Nitromethyl)cyclohexyl-essigsäure.

8. Benzyl-1-(nitromethyl)cyclohexyl-acetat.

9. Diphenylmethyl-1-(nitromethyl)cyclohexyl-acetat.

## Revendications

1. Procédé pour la synthèse de l'acide 1-(aminométhyl)-cyclohexyl-acétique et d'un sel acceptable du point de vue pharmaceutique de celui-ci, **caractérisé par** :
a) la transformation de l'ester alkylique de l'acide cyclohexylidène-acétique de formule (VI), dans laquelle R₂ représente un groupe alkyle en C₁₋₄, en l'ester alkylique de l'acide 1-(nitrométhyl)-cyclohexyl-acétique de formule (V), dans laquelle la signification de R₂ est telle que définie plus haut, avec du nitrométhane en présence d'une base, l'hydrolyse avec une solution méthanolique aqueuse d'hydroxyde de potasium et l'hydrogénation de l'acide 1-(nitrométhyl)-cyclohexyl-acétique de formule (IIa) obtenu dans un solvant, en présence d'un catalyseur et le cas échéant, la transformation du composé obtenu en un sel acceptable du point de vue pharmaceutique, ou bien : 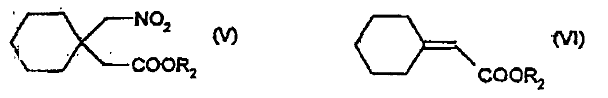 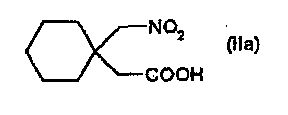
b) l'hydrolyse de l'ester alkylique de l'acide cyclohexylidène-acétique de formule (VI), dans laquelle R₂ représente un groupe alkyle en C₁₋₄, en l'acide cyclohexylidène-acétique de formule (IV) avec une solution méthanolique aqueuse d'hydroxyde de potassium, la réaction de l'acide obtenu de formule (IV) avec un réactif de formule R₁-X, dans laquelle R₁ représente un groupe benzyle, un groupe diphénylméthyle ou le cas échéant, leurs dérivés substitués par un groupe alkyle en C₁₋₄, ou alcoxy sur le noyau aromatique, et X représente un atome d'halogène, pour donner le dérivé intermédiaire de l'acide cyclohexylidène de formule (III), dans laquelle la signification de R₁ est telle que définie plus haut, la transformation de cet intermédiaire en dérivé de l'acide 1-(nitrométhyl)-cyclohexylacétique de formule (IIb), dans laquelle la signification de R₁ est telle que définie plus haut, avec du nitrométhane, et l'hydrogénation de ce dernier dans un solvant en présence d'un catalyseur et le cas échéant, la transformation du composé obtenu en un sel acceptable du point de vue pharmaceutique :

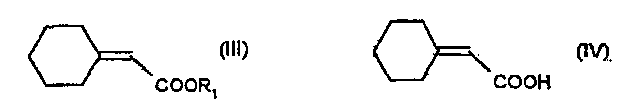

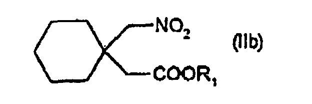

2. Procédé b) selon la revendication 1, **caractérisé par** l'utilisation d'halogénure de benzyle en tant que réactif de formule R₁-X.

3. Procédé b) selon la revendication 1, **caractérisé par** l'utilisation d'halogénure de diphénylméthyle en tant que réactif de formule R₁-X.

4. Procédé selon les revendications 1 à 3, **caractérisé par** la réalisation de l'hydrogénation dans un solvant organique inerte.

5. Procédé selon les revendications 1 à 3, **caractérisé par** l'utilisation de palladium sur du carbone activé en tant que catalyseur.

6. Nouveaux composés de formule (II), dans laquelle R représente un atome d'hydrogène, un groupe benzyle, diphénylméthyle ou le cas échéant, leurs dérivés substitués par un groupe alkyle en C₁₋₄ ou alcoxy sur le noyau aromatique.

7. Acide 1-(nitrométhyl)-cyclohexyl-acétique.

8. 1-(Nitrométhyl)-cyclohexyl-acétate de benzyle.

9. 1-(Nitrométhyl-cyclohexyl-acétate de diphénylméthyle.
